# EUROPEAN PATENT SPECIFICATION

(11) **EP 3 941 439 B1**
(45) Date of publication and mention of the grant of the patent: **29.07.2026**
(21) Application number: 20730723.2
(22) Date of filing: 08.05.2020
(51) Int. Cl.: A61K 9/107, A61K 9/51, A61K 9/19, A61K 47/02, A61K 47/08, A61K 47/10, A61K 47/12, A61K 47/26, A61K 47/32, A61K 47/34, A61K 31/05, A61K 31/137, A61K 31/496, A61P 3/04, A61P 25/22, A61P 25/24, A61P 29/00, A61P 31/06, A61P 31/10, A61P 31/12, A61P 33/06

(54) **POLYMER-LIPID NANOCOMPLEX FOR ENHANCED AQUEOUS SOLUBILISATION AND ABSORPTION OF HYDROPHOBIC ACTIVE COMPOUNDS**
POLYMER-LIPID-NANOKOMPLEX ZUR VERBESSERTEN WÄSSRIGEN SOLUBILISIERUNG UND ABSORPTION VON HYDROPHOBEN WIRKSTOFFVERBINDUNGEN
NANOCOMPLEXE POLYMÈRE-LIPIDE POUR SOLUBILISATION AQUEUSE AMÉLIORÉE ET ABSORPTION DE COMPOSÉS ACTIFS HYDROPHOBES

(30) Priority: 14.05.2019 ZA 201902992
(43) Date of publication of application: 26.01.2022
(73) Proprietor: Council for Scientific and Industrial Research, 0001 Pretoria (ZA)
(72) Inventor: NKUNA, Tshepo Patric, 0152 Pretoria (ZA); KALOMBO, Michel Lonji, 0081 Pretoria (ZA)
(74) Representative: Symbiosis IP Limited
(86) International application number: PCT/IB2020/054370
(87) International publication number: WO 2020/229971

(56) References cited:
- WO-A1-2009/105792
- WO-A1-2019/016819
- WO-A1-2019/055539
- US-A1- 2017 087 096
- DURGARAMANI SIVADASAN ET AL: "Formulation and Characterization of Solid Lipid Nanoparticles of Rifampicin", ERCIYES TIP DERGISI/ERCIYES MEDICAL JOURNAL, vol. 35, no. 1, 11 March 2013 (2013-03-11), pages 1 - 5, XP055720307, ISSN: 1300-199X, DOI: 10.5152/etd.2013.01
- JOSHY K S ET AL: "Evaluation of in-vitro cytotoxicity and cellular uptake efficiency of zidovudine-loaded solid lipid nanoparticles modified with Aloe Vera in glioma cells", MATERIALS SCIENCE AND ENGINEERING C, ELSEVIER SCIENCE S.A, CH, vol. 66, 19 March 2016 (2016-03-19), pages 40 - 50, XP029541420, ISSN: 0928-4931, DOI: 10.1016/J.MSEC.2016.03.031
- TORGE AFRA ET AL: "Ciprofloxacin-loaded lipid-core nanocapsules as mucus penetrating drug delivery system intended for the treatment of bacterial infections in cystic fibrosis", INTERNATIONAL JOURNAL OF PHARMACEUTICS, ELSEVIER, NL, vol. 527, no. 1, 10 May 2017 (2017-05-10), pages 92 - 102, XP085064661, ISSN: 0378-5173, DOI: 10.1016/J.IJPHARM.2017.05.013

## Description

### FIELD OF THE INVENTION

The current invention relates to a process for producing a polymer-lipid nanocomplex, to a method for enhancing aqueous solubilisation and absorption of hydrophobic active compounds by formulating them according to the process for producing such a nanocomplex, to a polymer-lipid nanocomplex obtained by such a process, and to such a polymer-lipid nanocomplex for use in a method of treatment or prophylaxis of certain disorders.

### BACKGROUND OF THE INVENTION

It is estimated that more than 40% of new chemical entities (NCEs) approved by the FDA in the pharmaceutical industry and nearly 90% of the lead compounds coming in the developmental pipeline consist of water-poorly soluble active compounds.

Poor water solubility or hydrophobicity is a prime compound property among the key physicochemical parameters that are used in the early screening process for best pharmaceutical candidates.

Drugs suffering from poor water solubility, present subsequently low permeability, rapid metabolism, high protein bound by plasma proteins for elimination from the body, a huge dose-to-solubility ratio, thus low bioavailability. The therapeutic levels are achieved with large quantities of the drug that may result in poor safety and tolerability, thus an emergence of drug resistance and undesirable side effects [1, 2].

The hydrophobicity of a compound is usually expressed by its ability to partition between an organic solvent (octanol) and water, also known as the partition coefficient (Log P). The positive value of Log P is ranked as hydrophobic compounds and highly hydrophobic as the values reached 10, translating in solubility below 10-4 as depicted in Table 1 suggested by the FDA [1]. This is a real challenge for the pharmaceutical industry because if the actives transit undissolved in the gastrointestinal tract (GIT), they will not be able to reach the target site for their intended effects in the body; hence they will remain useless compounds. Furthermore, hydrophobic drugs often require an intake of fat-rich food to facilitate their absorption, leading to erratic absorption with large variability in the inter-and-intrapatient dose response.

**Table 1: USP solubility description**

| **Descriptive term** | **Parts of solvent required for 1 part of solute** |
|---|---|
| Very soluble | <1 |
| Freely soluble | From 1 to 10 |
| Soluble | From 10 to 30 |
| Sparingly soluble | From 30 to 100 |
| Slightly soluble | From 100 to 1000 |
| Very slightly soluble | From 1000 to 10,000 |
| Practically insoluble or insoluble | 10,000 and over |

Another aspect to always consider during drug development is the categorization of drugs as given by the Biopharmaceutical Classification system (BCS) in Table 2 [3]. This system assists in the prediction of the drug behavior using concomitantly the three parameters of drug including the solubility, dissolution and intestinal permeability. These are important parameters that will assist reaching preferred levels of drug in the systemic circulation following an oral administration for desired pharmacological response. The BCS is therefore an effective tool that is extensively used in drug development processes [3].

**Table 2: Biopharmaceutical drugs classification system**

| Class | Solubility | Permeability |
|---|---|---|
| I | High | High |
| II | Low | High |
| III | High | Low |
| IV | Low | Low |

All these types of drugs pose inherent challenges, and Class IV is usually considered to be outside of the so-called "drugable space", but if proven to be efficacious, it can be formulated to overcome its dual challenges of low solubility and low permeability.

Several strategies have been used in the pharmaceutical industry to enhance drug solubility. They include: physical modifications (e.g. size reduction), chemical modifications (e.g. polymer-drug conjugation) and miscellaneous methods including supercritical fluid micronisation. Vimalson et al [1] has extensively reviewed the various techniques to achieve these modifications. It is worth mentioning that most of the physical modifications aimed at reducing the size, the crystallinity and altering the morphological habit of drug particles in order to increase the solubility.

This mechanism of improving drug solubility by physical means is well described by Noyes-Whitney equation [4] expressing the increase of the dissolution rate of the API following the reduction of the active pharmaceutical ingredient (API) particle size to submicron range, thus the shrinking of the effective boundary layer thickness. However, it is directly proportional to the particle surface area. Emulsions-based strategies are equally extensively used in the pharmaceutical industry in order to increase the drug solubility and dissolution rate. In this case, a nanoemulsion or microemulsion is used to encapsulate the hydrophobic drug, subsequently rendering it soluble in aqueous solutions. Depending on the power of solvency of the emulsions, only hydrophobic actives with a narrow range of Log P can be solubilised in these solutions.

There is therefore a need for a microemulsion system that is able to facilitate the solubilisation of a broad range of actives from highly refractory hydrophobic actives (i.e. Log P of 9) to lesser hydrophobic compounds (with a Log P below 3).

Furthermore, it is not sufficient to just increase the dissolution rate in the GIT of orally administered actives in order to increase their bioavailability in the systemic circulation. Strategies aimed at improving their permeability through the epithelial linings of the intestine and ensuring a prolonged residence time in the blood to achieve high levels of drugs are equally needed. WO 2009/105792 A1 discloses a process for the production of nanoparticle carriers for drug delivery, said nanoparticles being produced by preparing a double emulsion of water-oil-water including a polymer which forms the basis of the nanoparticle carrier, blending the drug to be delivered into one of the emulsion phases, doping either the oil-phase or the outer-water phase with a carbohydrate, and spray-drying the emulsion to form nanoparticles of a narrow particle size distribution of 100 nm to 1000 nm.

### SUMMARY OF THE INVENTION

According to a first aspect of the invention, there is provided a process for producing a polymer-lipid nanocomplex comprising the steps of:
I. mixing at least one hydrophobic active compound positioned on the Log P positive scale selected from Log P 2 to Log P 9 of the partition coefficient scale, a fatty acid consisting of any one or more of stearic acid, palmitic acid and lauric acid dissolved in a polar solvent consisting of any one of ethanol, acetone, and a blend of ethanol and acetone, and a surfactant having a Hydrophile-Lipophile Balance (HLB) value of greater than 10 comprising polysorbate 80, to form an organic phase;
II. optionally heating the organic phase;
III. dispensing the organic phase into an aqueous solution consisting of an aqueous mixture of polyvinyl alcohol (PVA) and polyethylene glycol (PEG) to form a microemulsion; and
IV. stabilising the microemulsion in a phosphate buffer at about 0°C to form a nanoprecipitate of the polymer-lipid nanocomplex.

The process may further comprise a final step of drying the nanoprecipitate to produce a free flowing polymer-lipid nanocomplex powder either by freeze drying or by spray drying.

The process may further comprise mixing an organic carboxylic acid with the organic phase.

The process may further comprise dissolving at least one biocompatible and biodegradable polymer or copolymer suitable for use in drug delivery, poly(lactic-co-glycolic acid) or PLGA, or polylactic acid, polyglycolic acid, or poly ε-caprolactone, into the polar solvent with the fatty acid for mixing with the at least one hydrophobic active compound and the surfactant to form the organic phase.

The at least one fatty acid preferably consists of stearic acid.

The polar solvent preferably consists of acetone.

The organic carboxylic acid may comprise at least one weak acid. For example, the weak acid may include any one or more of those approved for human consumption comprising acetic acid, lactic acid, citric acid, or phosphoric acid. Preferably the weak acid is acetic acid.

The surfactant is polysorbate 80, also known as Tween 80^{®}.

In particular, the process may comprise:
a) dissolving at least one fatty acid as claimed in a polar solvent as claimed to form a fatty acid solution;
b) dissolving at least one hydrophobic active compound as claimed in the fatty acid solution;
c) adding drop-wise, a surfactant as claimed to form an organic phase;
d) optionally heating the organic phase;
e) dispensing the organic phase into an aqueous solution as claimed while stirring to form a microemulsion; and
f) nanoprecipitating the microemulsion by adding a phosphate buffer at 0°C while stirring to form the polymer-lipid nanocomplex.

The process may further comprise an additional step of drying the nanoprecipitate to produce a free flowing polymer-lipid nanocomplex powder either by freeze drying or by spray drying.

The process may further comprise, at step a), dissolving PLGA, or alternatively, any biocompatible and biodegradable polymer suitable for use in drug delivery, including polylactic acid, polyglycolic acid, or poly ε-caprolactone, into the polar solvent with the fatty acid.

The process may further comprise, at step c), adding drop-wise, an organic carboxylic acid with the surfactant.

The process may further comprise in step e) heating while stirring to form the microemulsion. The heating steps may be performed at from between about 40 °C to 50 °C, preferably 40 °C.

The phosphate buffer may comprise a pH of from about 7.2 to about 7.6, more preferably, about 7.4 at 0°C.

The nanoprecipitation of the microemulsion may be performed by adding the microemulsion to the phosphate buffer solution at a ratio of about 1:1. It is to be appreciated that a variety of factors influence the optimum ratio of microemulsion to buffer, including drug loading, stability of the formulation, including during the drying process, and the like.

The freeze drying may be performed following an initial snap -freezing step in liquid nitrogen.

The spray drying may be performed using a spray dryer such as the Top bench Buchi-B290. In particular, such spray drying may be performed with the following set of parameters;
- Inlet temperature: about 90 to 110°C
- Outlet temperature: about 60 °C
- Feeding rate: 2% (mL/min)
- Atomizing pressure: 6-7 bar
- Aspiration vacuum set at 100 %.

It is to be appreciated that the inlet temperature should be high enough to evaporate both the polar (water) and nonpolar (organic) solvents without degrading any compounds in the formulation, and that the range provided is one embodiment of the invention and may be modified by those skilled in the art.

It is further to be appreciated that the outlet temperature is affected by the room temperature of the lab in which the apparatus is situated and, apart from requiring that the outlet temperature is above 60 °C in order to obtain a dry, free flowing powder, the specific termperature may vary. The outlet temperature is equally governed by the liquid feeding rate, the inlet temperature and thermal exchange efficiency between the droplets and the drying hot air.

The dried polymer-lipid nanocomplex size may range from between about 150 nm to 500 nm.

The liquid polymer-lipid nanocomplex size may range from between about 70 nm to 150 nm.

The dried polymer-lipid nanocomplex may comprise a polydispersity index (PDI) of 0.3 or less.

According to a further aspect of the invention, there is provided a method for enhancing aqueous solubilisation and absorption of at least one hydrophobic active compound comprising formulating the at least one hydrophobic active compound using the process of the invention.

The at least one hydrophobic active compound comprises any hydrophobic active compound positioned on the Log P positive scale (Log P 2 to Log P 9) of the partition coefficient scale. For example, the at least one hydrophobic active compound may comprise any one or more of antituberculosis drugs, antimalarials, and phytochemicals with antifungal, antiviral, antianxiety, antiobesity, antidepressant and/or analgesic properties.

In particular, the at least one hydrophobic active compound may comprise an antimalarial agent, and more specifically Lumefantrine.

Further in particular, the at least one hydrophobic active compound may comprise a phytochemical, and more specifically Cannabidiol (CBD).

Further in particular, the at least one hydrophobic active compound may comprise an anti-tuberculosis drug, and more specifically Rifampicin.

For example, the at least one hydrophobic active compound formulated according to the invention may be for administration through various routes including topical, sub-cutaneous, intravenous, intramuscular, inhalable, nasal, sub-lingual, buccal, rectal, or ophthalmic.

According to a further embodiment of the invention, there is provided a polymer-lipid nanocomplex obtained by the process of the invention.

According to a further embodiment of the invention, there is provided such a polymer-lipid nanocomplex of the invention for use in a method of treatment or prophylaxis of tuberculosis, malaria, fungal infections, viral infections, anxiety, obesity, depression and/or pain in a subject.

The administration may be through various routes including topical, sub-cutaneous, intravenous, intramuscular, nasal, sub-lingual, inhalable, buccal, rectal or ophthalmic.

### BRIEF DESCRIPTION OF THE DRAWINGS

- **Figure 1**: shows a size distribution and the PDI of the LUM microemulsion;
- **Figure 2**: shows a DSC Thermal graph for the nanocomplexed LUM as compared to free drug;
- **Figure 3**: shows a graph of the direct comparison for delivery of LUM compared to nanocomplexed LUM, dosed orally at an equivalent 20mpk of LUM in male Balb/c mice;
- **Figure 4**: shows a graph of the *in vivo* efficacy results of nanocomplexed LUM in a malaria-infected NSG mice model;
- **Figure 5**: shows a graph of the *in vivo* dose-response relationship of LUM- loaded nanocomplexes;
- **Figure 6**: shows a size distribution and the PDI of the CBD microemulsion;
- **Figure 7**: shows a size distribution and the PDI of the CBD powder;
- **Figure 8**: shows a size distribution, PDI, zeta potential, yield and the drug loading of the RIF powder;
- **Figure 9**: shows the morphology (SEM) of the RIF powder; and
- **Figure 10**: shows a graph of the *in vitro* release profile of RIF-loaded nanocomplexes.

### DETAILED DESCRIPTION OF THE INVENTION

The present invention relates to a process for producing a polymer-lipid nanocomplex, to a method for enhancing aqueous solubilisation and absorption of hydrophobic active compounds by formulating them according to the process for producing such a nanocomplex, to a polymer-lipid nanocomplex obtained by such a process, and to such a polymer-lipid nanocomplex for use in a method of treatment or prophylaxis of certain disorders.

Although the applicant has shown that several hydrophobic active compounds having Log P values selected from Log P 2 to Log P 9, including antituberculosis drugs, antimalarials, and phytochemicals with antifungal, antiviral, antianxiety, antiobesity, antidepressant and/or analgesic properties, are able to be successfully incorporated in this polymer-lipid nanocomplex, the invention will be described in terms of three exemplary examples; an antimalarial drug, Lumefantrine, a phytochemical, Cannabidiol, and an anti-tuberculosis (TB) drug, Rifampicin.

This is a simple, easy-to-scale up process that would merely require conventional pharmaceutical equipment for production on a commercial scale.

The exemplary examples below are for illustrative purposes and should in no way be construed as limiting in any way the scope of the invention.

### EXAMPLE ONE

### Lumefantrine (Log P 9) - Antimalarial drug

### 1. Background

Lumefantrine is a water insoluble (Log P of 9) racemic mixture of synthetic fluorene derivatives with the chemical structure as shown in Formula 1.

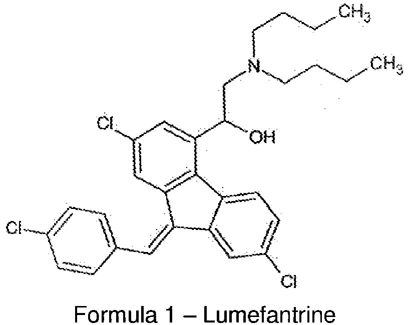

Lumefantrine is used for the treatment of acute uncomplicated malaria caused by a protozoan parasite, *Plasmodium falciparum (P. falciparum).* It is administered in combination with an artemisinin methyl ether derivative, artemether, to potentiate efficacy. Lumefantrine is a blood schizonticide active against the erythrocyte stage of uncomplicated *P. falciparum* infections and has a half-life (T1/2) of 4 - 5 days. Although efficacious, it possesses some limitations that hinder maximum efficacy. It has low and slow absorption into the circulatory system which leads to relatively low bioavailability, it is extensively metabolized in the liver and, due to its high lipophilicity, it is highly protein bound (99.7%) upon entry into the blood. These limitations therefore result in a longer period of time (Tmax) for Lumefantrine to reach peak concentrations; it takes about 6 hours for orally administered Lumefantrine to reach the highest concentration (Cmax) of 7µg/mL in the blood.

As indicated above, Lumefantrine has delayed absorption, which negatively impacts its bioavailability. Its insolubility in polar media (0.002% w/v) results in it being highly metabolised by enzymes of the liver and it is significantly bound by plasma proteins upon crossing the intestinal tract into the blood.

### 2. Methods and Materials

200 - 300 mg Lumefantrine (LUM) was dissolved in a solution of stearic acid dissolved in a polar aprotic solvent, acetone, to which about 50 - 100 µl of an organic carboxylic acid, acetic acid, and about 50 - 100 µl of a surfactant with high Hydrophile-Lipophile Balance (HLB) value, polysorbate 80 or Tween 80^{®}, were added to speed up the dissolution of LUM resulting into the oil phase (internal) of the emulsion.

The organic phase of LUM was first heated up to 40°C, then rapidly dispensed into an aqueous solution of an aqueous mixture of hydrophilic polymers, polyvinyl alcohol (PVA) and polyethylene glycol (i.e. PEG 4000), while moderately stirring on a magnetic hot plate.

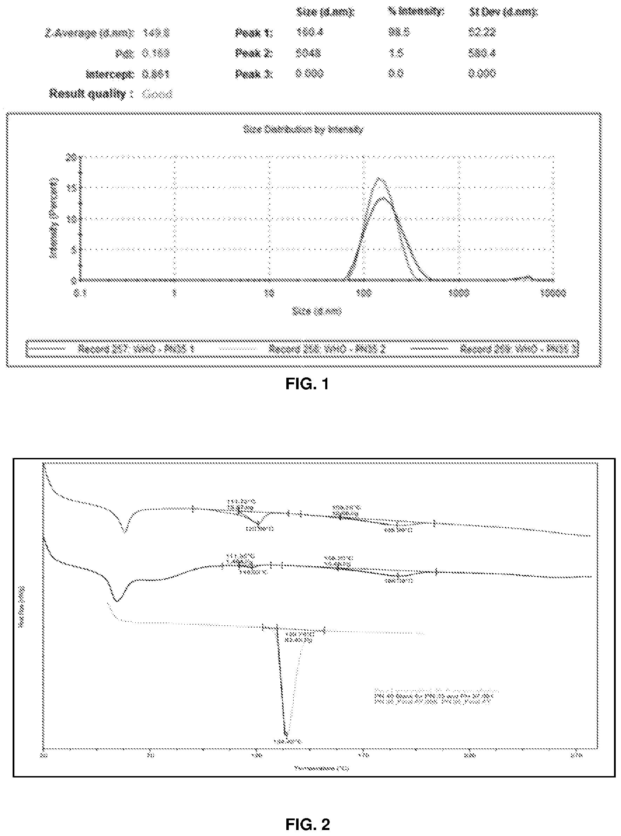

The microemulsion obtained has a very narrow size distribution as shown in Figure 1. The emulsion is further stabilized by immediately transferring the emulsion into a high volume (volume ratio 1:1) of an icy phosphate buffer solution (pH 7.4) at 0°C while stirring. The resulting suspo-emulsion was subsequently dried to produce a free flowing powder either by freeze drying following a snap freezing step in liquid nitrogen or by spray drying using a Top bench Buchi-B290 spray dryer with the following set of parameters:
- Inlet temperature: 100°C
- Outlet temperature: 60°C
- Feeding rate: 2% (mL/min)
- Atomizing pressure: 6-7 bar
- Aspiration vacuum set at 100%.

### 3. Results

The process resulted in the production of a free flowing powder of encapsulated LUM having a payload efficiency of circa 25% w/w, and 90% of nanoparticle sizes ranging from 200 to 300 nm, with a polydispersity index (PDI) of 0.3 and below.

The nanocomplexes produced by the process were readily re-dispersed in water at a concentration of 25 mg/ml without any visible sign of precipitation and the nanosuspension was still stable.

Results showed significantly higher amounts of suspended drug in water as compared to the drug's inherent water solubility of just 0.002% w/v, thus translating in more than 10 000-fold improvement that was achieved.

Due to its ability to form a clear solution when re-dispersed in aqueous media, the LUM-loaded nanocomplexes can be administered via a number of routes, including orally or intravenously.

The thermal analysis (differential scanning calorimetry or DSC) of the free flowing powder showed a disappearance of the melting point peak of LUM that could be interpreted as a conversion from its crystalline morphology to amorphous structure (see Figure 2). This is beneficial as it indicates a rapid dissolution rate of the drug.

### Pharmacokinetic and Pharmacodynamic evaluation of nanoformulated LUM (PN35)

LUM was reformulated into the lipid-polymer nanocomplex for delivery with the aim of improving its bioavailability and enhancing its therapeutic efficacy *in vivo.*

This experiment demonstrated pharmacokinetic parameters (i.e. Cmax) 10-fold greater than the control of un-formulated LUM, with a distinctive prolonged tailing curve above the minimum inhibitory concentration (MIC) for several hours. It shows a fast absorption of LUM following oral administration depicted by a short Tmax.

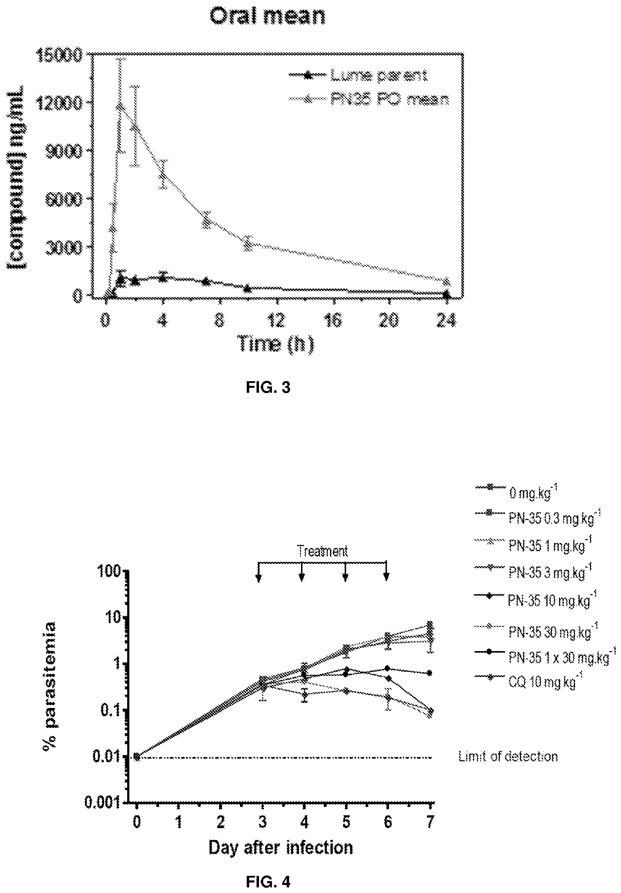

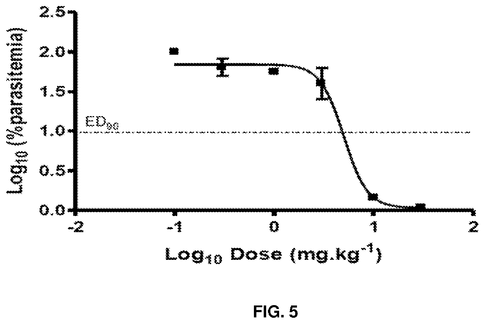

Enhanced absorption was observed in animal studies a few minutes after oral administration of the nanocomplexed LUM resuspended in water. The highest plasma concentrations ever reported of 11.5 mg/mL were obtained in less than 1 hour, thus minimizing the lag-time for LUM absorption. Furthermore, the reported effective dose (ED90) of LUM to clear 90% of parasitaemia in the blood was significantly reduced from 12.01 mg/kg to 5.01 mg/kg (see Figures 3, 4 and 5).

### EXAMPLE TWO

### Cannabidiol (Log P 6) - Phytochemical analgesic drug

### 1. Background

Cannabidiol (CBD) is a naturally occurring chemical compound or phytochemical that is found in cannabis plants. It is one of the 113 cannabinoid compound extracts from cannabis plants and it is the major phytocannabinoid compound which makes up 40% of the total plant extracts. It belongs to the cannabinoid drug class and can be administered through inhalation with bioavailabilities ranging from 11 - 45% and orally with only 13 - 19% bioavailability. The extract (Formula 2) can be administered in a solution form for oral administration or as an additive in food preparation. It has major medicinal benefits to humans including pain and inflammation relief, anxiety management, seizure control and also has antioxidant properties.

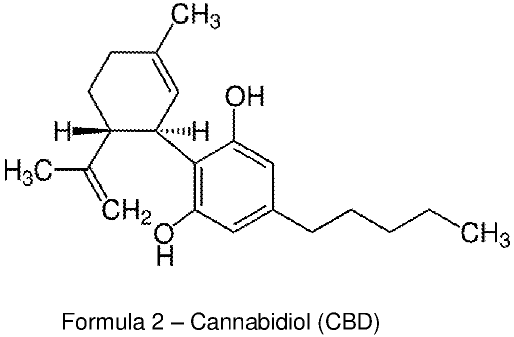

Although CBD is derived from the cannabis plant, which is well known for its psychotropic effects when used recreationally, CBD does not induce psychotropic effects like its co-synthesized cannabis extract, Tetrahydrocannabinol (THC) which is the principal psychoactive constituent in the plant. It has also been hypothesized that it may act as antagonist for THC since they bind to the same receptors found in the central and peripheral nervous system of the human body.

CBD research studies across the world have shown many significant health benefits including pain relief capabilities and reversed inflammatory effects. In addition, combination of CBD with THC in several human studies has shown effective treatment for pain in multiple sclerosis and arthritis. In recent studies with cancer patients, elevated reduction of cancer-related symptoms were also observed. It improves heart health through its anti-oxidative properties, has antitumor effects, reduces acne prevalence, and has antipsychotic effects and good neuroprotective properties. Treatment with CBD in some studies has suggested the prevention of diabetes and also has high reduction of anxiety and depression. It has also been used to treat patients with substance (drugs etc.) abuse. Although the extract has proven to be beneficial in treating numerous ailments, it also induces a few undesirable immune responses. Side effects include nausea, vomiting, drowsiness, dizziness, and diarrhea, changes in appetite, anxiety, depression and psychosis. The exact mechanism for its beneficial health effects is not clear, but cannabidiol seems to prevent the breakdown of a chemical in the brain that affects pain, mood, and mental function. Preventing the breakdown of this chemical and increasing its levels in the blood seems to reduce psychotic symptoms associated with conditions such as schizophrenia.

The extract is a water insoluble (0.0126 mg/mL), colourless crystalline powder and it is soluble inevarious organic solvents. CBD is known to be non-psychoactive, although it is highly insoluble in water, thus impeding absorption, and is also subjected to significant first-pass metabolism. Both these properties are major limitations to treatment outcomes and also contribute to its low bioavailability when orally administered.

### 2. Methods and Materials

### Liquid formulation

The CBD (10 to 20 mg) was dissolved in stearic acid and a co-solution of acetone/ethanol, and then 10 to 20 µl of a surfactant with a high HLB value above 10 (Tween 80^{®}) was added to assist in the formation of the oil phase droplets. It is also possible to optionally dissolve PLGA, or alternatively, any biocompatible and biodegradable polymer suitable for use in drug delivery, including polylactic acid, polyglycolic acid, or poly ε-caprolactone, in the stearic acid and acetone/ethanol co-solution to further improve stability of the hydrophobic active in the inner matrix of the microemulsion.

To form the liquid microemulsion, the organic phase was rapidly dispensed into an aqueous solution of an aqueous mixture of hydrophilic polymers, polyvinyl alcohol (PVA) and polyethylene glycol (i.e. PEG 4000). The organic phase may optionally first be heated to about 40°C, then, when dispensed into the aqueous mixture of hydrophilic polymers, may be moderately stirred using a magnetic hot plate at about 40°C, resulting in a stable microemulsion with reproducible droplet size and size distribution.

### Powder formulation

200 - 300 mg of CBD was dissolved in a solution of stearic acid dissolved in a polar aprotic solvent, acetone, to which 50 - 100 µl of an organic carboxylic acid, acetic acid, and 10-20 µl of a surfactant with Hydrophile-Lipophile Balance (HLB) value greater than 10, polysorbate 80 or Tween 80^{®}, were added, resulting into the oil phase (internal) of the emulsion.

The dissolved CBD oil phase was then rapidly dispensed into an aqueous solution of an aqueous mixture of hydrophilic polymers, polyvinyl alcohol (PVA) and polyethylene glycol (i.e. PEG 4000), while moderately stirring. The dissolved CBD oil phase may optionally be heated to about 40°C before adding the aqueous mixture of hydrophilic polymers, and the moderate stirring may be performed at about 40°C on a magnetic hot plate.

The CBD emulsion was further stabilized through incorporation of a high volume (volume ratio 1:1) of an icy phosphate buffer solution (PBS) pH 7.4 at 0°C while stirring.

The stabilized emulsion was subsequently dried to produce a free flowing powder either by freeze drying following a snap freezing step in liquid nitrogen or by spray drying using a Top bench Buchi-B290 spray dryer under the following conditions:
- Inlet temperature: 100°C
- Outlet temperature: 60°C
- Feeding rate: with the pump knob set at 2
- Atomizing pressure: 6-7 bar
- Aspiration vacuum set at 100%.

### 3. Results

### Liquid formulation

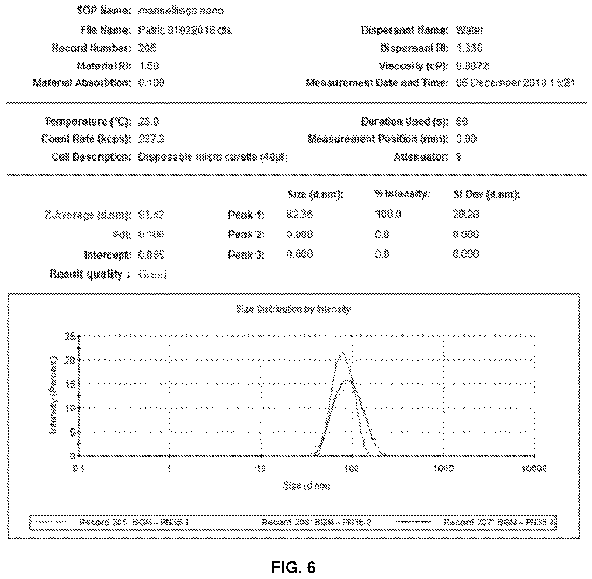

A water based microemulsion system (>95% water) incorporating CBD was successfully developed using an oil-in-water single emulsion nanoprecipitation technique. The emulsion incorporated 2 mg of CBD in every 1mL of water (more than 50 X improvement) with homogeneous size distributions and oil droplet sizes ranging from 70 nm to 150 nm (Figure 6) depending on the CBD concentrations and solvent to water ratios. All the formulations incorporating CBD from low to high concentrations have been stable for over 6 months and all retain their initial sizes and size distributions with less that 2% (± 3 nm) of size variations.

### Powder formulation

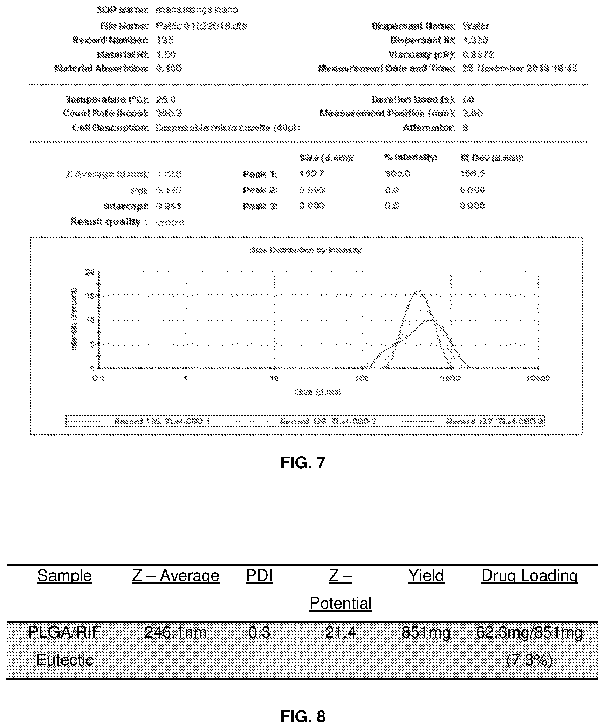

The CBD powder is water soluble with size distributions ranging from 200 nm to 460 nm (Figure 7). The stability of the CBD powder was investigated at room temperature and during refrigeration at between about 4-8 °C and was found to be stable over six months.

### EXAMPLE THREE

### Rifampicin (Log P 3.85) - Anti-Tuberculosis drug

### 1. Background

Today, almost one-third of the world population is infected with latent tuberculosis (TB), resulting in the death of two million people every year. TB is caused by a bacillus bacterium, *Mycobacterium tuberculosis* (M.TB) and it usually attacks the lungs, but other body systems can also get affected including the central nervous system, the lymphatic system, bones and skin. TB is an airborne disease that usually spreads through the air from infected people by cough, sneeze or spit. Infection occurs upon inhalation of the bacterium which is engulfed by alveolar macrophages and throughout the disease progression, the bacterium remains primarily within the macrophages. Here, the bacterium is able to remain in a latent state or multiply and disseminate to other sites and/or be transmitted to other individuals. Current treatment is strenuous, requiring patients to take high drug doses of a combination therapy (Isoniazid, Rifampicin, Pyrazinamide and Ethambutol) per day over a long period of time extending up to at least 18 months often with severe drug related side effect manifestations. As a result, patient compliance to the treatment regimen is low and this has significantly contributed to the emergence of drug resistant M.TB strains. Multidrug resistant-TB (MDR-TB) strains are defined as strains resistant to the frontline drugs, Isoniazid and Rifampicin.

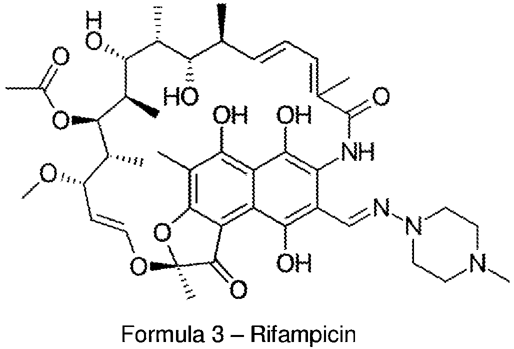

In 2008-2009, the highest ever number of MDR-TB cases were reported to WHO, about half a million new MDR-TB cases emerging annually. Drug-resistant TB has a higher mortality rate, and is significantly more difficult and costly to treat than drug-susceptible TB. In South Africa, the recommended MDR-TB treatment regimen consists of a 6-month intensive phase (requiring hospitalization for the entire intensive phase), with daily administration of one injectable drug, i.e. kanamycin, together with at least four pills (Ethionamide, Ofloxacin, Terizidone or Cycloserine), followed by an 18-month continuation phase of daily intake of the four pills. These drugs exhibit significant toxicity, e.g. kanamycin is associated with severe dose dependent auditory and renal damage. Most patients complete treatment with permanent hearing loss. Moreover, failure to adhere to the treatment may lead to a development of a more multi-drug resistant strain, extremely-drug resistant (XDR-TB). This strain is even more difficult to treat with the currently available antibiotics. XDR-TB treatment consists of Capreomycin injection and orally administered Ethionamide, Para-amino Salicylic acid, Moxifloxacin, Terizidone, Pyrazinamide and Clofazimine.The length of the intensive phase is guided by culture conversion but is recommended to be at least 6 months, and the continuation phase recommended being at least 18 months. Again these drugs exhibit severe side effects and combined with the nature of the treatment regimen make patient compliance difficult. Patients also face the prospect of development of further resistance beyond XDR-TB, dubbed Totally Drug Resistant-TB (TDR-TB) which is currently considered incurable.

The project focuses on encapsulation of Rifampicin in biodegradable/biocompatible polymers and lipids. This will improve absorption, protect the drug from the first pass metabolism and subsequently improve the drug bioavailability with an extended plasma half-life.

### 2. Methods and Materials

200 - 300 mg Rifampicin (RIF) was dissolved in a solution of stearic acid dissolved in a polar aprotic solvent, acetone. Optionally, PLGA, or alternatively, any biocompatible and biodegradable polymer suitable for use in drug delivery, including polylactic acid, polyglycolic acid, or poly ε-caprolactone, may further be dissolved in the polar aprotic solvent to improve the stability of the hydrophobic RIF in the inner matrix of the microemulsion. About 10-20 µl droplets of a surfactant with a Hydrophile-Lipophile Balance (HLB) value of greater than 10, Tween 80^{®}, were added to speed up the dissolution of RIF resulting into the oil phase (internal) of the emulsion. The dissolved RIF was then added to a pool of excipients composed of PVA and PEG to form the emulsion. The resulting emulsion was immediately transferred into a cold PBS (pH 7.4) buffer solution at 0 °C at a 1:1 ratio to further stabilise the emulsion. The latter was subsequently dried to produce a free flowing powder either by freeze drying following a snap freezing step in liquid nitrogen or by spray drying using a Top bench Buchi-B290 spray dryer with the following set of parameters:
- Inlet temperature: 100°C
- Outlet temperature: 60°C
- Feeding rate: with the pump knob set at 2
- Atomizing pressure: 6-7 bar
- Aspiration vacuum set at 100%.

### 3. Results

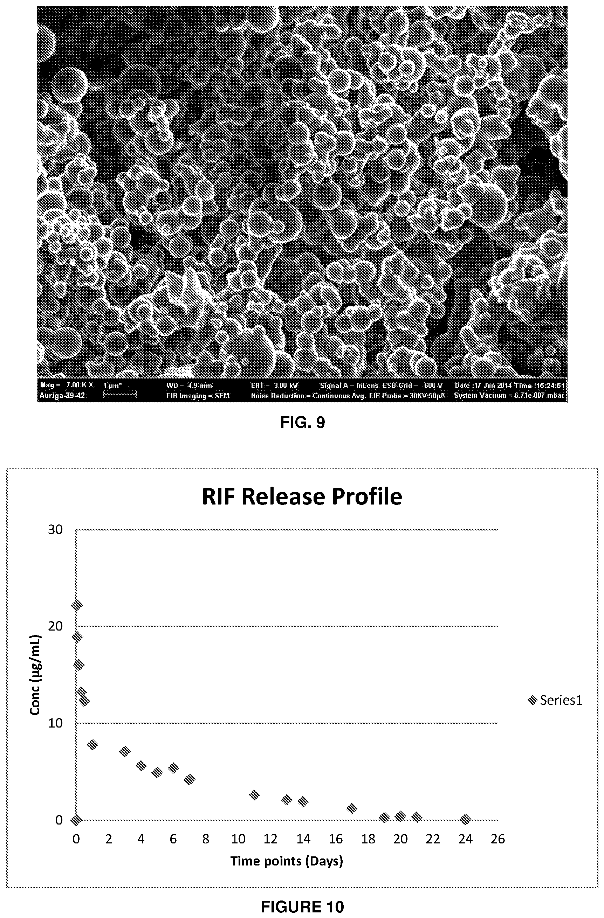

The hydrodynamic size averaged about 246 nm with good distribution below 0.3 (Figure 8). The morphology was observed through a scanning electron microscope (SEM) and resulted in spherical particles (Figure 9).

The encapsulation efficiency was above 75% w/w with a solid recovery yield of over 90%. The drug loading of RIF was between 7 and 10%. The latter relatively low drug loading of a hydrophobic drug such as RIF might result from the tendency of RIF to precipitate outside of the polymeric shell.

The *in vitro* release studies were carried out for 25 days in a shaking water bath set at 37°C and 100 rpm. A solution of 1 mg/mL, 40 mg of PLGA-RIF NP suspended in 40 mL of PBS pH 7.4, was prepared, all samples were in triplicates.

Controlled release of RIF was observed for the whole period of 25 days. An initial burst release in just a few minutes was observed, reaching the highest concentrations over 20 µg/mL, followed by low but sustained levels of release for the entire study (Figure 10).

### REFERENCES

1. D. C. Vimalson, S. Parimalakrishnan, N. S. Jeganathan, S. Anbazhagan. Techniques to Enhance Solubility of Hydrophobic Drugs: An Overview. Asian Journal of Pharmaceutics • Apr-Jun 2016 (Suppl) • 10 (2) | S67
2. S. Kalepua, V. Nekkantib. Insoluble drug delivery strategies: review of recent advances and business prospects. Acta Pharmaceutica Sinica B 2015;5(5):442-453
3. G.L. Amidon, H Lennernäs , VP Shah , JR Crison (1995). A theoretical basis for a biopharmaceutic drug classification: The correlation of in vitro drug product dissolution and in vivo bioavailability. Pharmaceutical Research, 12, 413-420
4. F. Kesisoglou, S. Panmai, Y.W. (2007). Nanosizing - Oral formulation development and biopharmaceutical evaluation. Advanced Drug Delivery Reviews, 59, 631-644

## Claims

1. A process for producing a polymer-lipid nanocomplex comprising the steps of:
I. mixing at least one hydrophobic active compound positioned on the Log P positive scale selected from Log P 2 to Log P 9 of the partition coefficient scale, a fatty acid consisting of any one or more of stearic acid, palmitic acid and lauric acid dissolved in a polar solvent consisting of any one of ethanol, acetone, and a blend of ethanol and acetone, and a surfactant having a Hydrophile-Lipophile Balance (HLB) value of greater than 10 comprising polysorbate 80, to form an organic phase;
II. optionally heating the organic phase;
III. dispensing the organic phase into an aqueous solution consisting of an aqueous mixture of polyvinyl alcohol (PVA) and polyethylene glycol (PEG) to form a microemulsion; and
IV. stabilising the microemulsion in a phosphate buffer at about 0°C to form a nanoprecipitate of the polymer-lipid nanocomplex.

2. The process according to claim 1, which further comprises a final step of drying the nanoprecipitate to produce a free flowing polymer-lipid nanocomplex powder either by freeze drying or by spray drying.

3. The process according to either claim 1 or 2, which further comprises mixing an organic carboxylic acid comprising acetic acid, lactic acid, citric acid, or phosphoric acid with the organic phase.

4. The process according to any one of claims 1 to 3, wherein the process further comprises dissolving at least one biodegradable and biocompatible polymer or copolymer, poly(lactic-co-glycolic acid) or PLGA, or polylactic acid, polyglycolic acid, or poly ε-caprolactone, into the polar solvent with the fatty acid for mixing with the at least one hydrophobic active compound and the surfactant to form the organic phase.

5. The process according to any one of claims 1 to 4, wherein in step IV, the precipitation of the microemulsion is performed by adding the microemulsion to the phosphate buffer solution at a ratio about 1:1.

6. A method for enhancing aqueous solubilisation and absorption of at least one hydrophobic active compound, comprising formulating the at least one hydrophobic active compound according to the process according to any one of claims 1 to 5.

7. A polymer-lipid nanocomplex obtained by the process according to any one of claims 1 to 5.

8. The polymer-lipid nanocomplex according to claim 7, the process according to any one of claims 1 to 5, or the method of claim 6, wherein the at least one hydrophobic active compound is the antimalarial agent Lumefantrine.

9. The polymer-lipid nanocomplex according to claim 7, the process according to any one of claims 1 to 5, or the method of claim 6, wherein the at least one hydrophobic active compound is a phytochemical from Cannabinoids, including Cannabidiol.

10. The polymer-lipid nanocomplex according to claim 7, the process according to any one of claims 1 to 5, or the method of claim 6, wherein the at least one hydrophobic active compound is the anti-tuberculosis drug Rifampicin.

11. The method according to claim 6, wherein the at least one hydrophobic active compound is formulated for administration through topical, sub-cutaneous, intravenous, intramuscular, nasal, sub-lingual, buccal, or ophthalmic routes.

12. The polymer-lipid nanocomplex according to claim 7 for use in a method of treatment or prophylaxis of tuberculosis, malaria, fungal infections, viral infections, anxiety, obesity, depression and/or pain in a subject.

## Patentansprüche

1. Prozess zum Herstellen eines Polymer-Lipid-Nanokomplexes, umfassend die folgenden Schritte:
I. Mischen von zumindest einer hydrophoben Wirkstoffverbindung, die auf der positiven Log-P-Skala ausgewählt aus Log P 2 bis Log P 9 der Verteilungskoeffizientenskala positioniert ist, einer Fettsäure, die aus einem beliebigen oder mehreren von Stearinsäure, Palmitinsäure und Laurinsäure besteht, die in einem polaren Lösungsmittel aufgelöst ist, das aus einem beliebigen von Ethanol, Aceton und einem Gemisch aus Ethanol und Aceton besteht, und einem Tensid mit einem Hydrophil-Lipophil-Gleichgewichts(HLB-)Wert größer als 10, umfassend Polysorbat 80, um eine organische Phase zu bilden;
II. optional Erhitzen der organischen Phase;
III. Abgeben der organischen Phase in eine wässrige Lösung, die aus einer wässrigen Mischung aus Polyvinylalkohol (PVA) und Polyethylenglykol (PEG) besteht, um eine Mikroemulsion zu bilden; und
IV. Stabilisieren der Mikroemulsion in einem Phosphatpuffer bei etwa 0 °C, um ein Nanopräzipitat des Polymer-Lipid-Nanokomplexes zu bilden.

2. Prozess nach Anspruch 1, der ferner einen finalen Schritt des Trocknens des Nanopräzipitats umfasst, um ein freifließendes Polymer-Lipid-Nanokomplexpulver entweder durch Gefriertrocknen oder durch Sprühtrocknen herzustellen.

3. Prozess nach Anspruch 1 oder 2, der ferner Mischen einer organischen Carbonsäure, umfassend Essigsäure, Milchsäure, Zitronensäure oder Phosphorsäure, mit der organischen Phase umfasst.

4. Prozess nach einem der Ansprüche 1 bis 3, wobei der Prozess ferner Auflösen von zumindest einem biologisch abbaubaren und biokompatiblen Polymer oder Copolymer, Poly(milch-co-glykolsäure) oder PLGA, oder Polymilchsäure, Polyglykolsäure oder Poly-ε-caprolacton in dem polaren Lösungsmittel mit der Fettsäure zum Mischen mit der zumindest einen hydrophoben Wirkstoffverbindung und dem Tensid umfasst, um die organische Phase zu bilden.

5. Prozess nach einem der Ansprüche 1 bis 4, wobei in Schritt IV die Präzipitation der Mikroemulsion durch Hinzufügen der Mikroemulsion zu der Phosphatpufferlösung in einem Verhältnis von etwa 1:1 durchgeführt wird.

6. Verfahren zum Verbessern von wässriger Solubilisierung und Absorption von zumindest einer hydrophoben Wirkstoffverbindung, umfassend Formulieren der zumindest einen hydrophoben Wirkstoffverbindung nach dem Prozess nach einem der Ansprüche 1 bis 5.

7. Polymer-Lipid-Nanokomplex, der durch den Prozess nach einem der Ansprüche 1 bis 5 erhalten wird.

8. Polymer-Lipid-Nanokomplex nach Anspruch 7, Prozess nach einem der Ansprüche 1 bis 5 oder Verfahren nach Anspruch 6, wobei die zumindest eine hydrophobe Wirkstoffverbindung das Antimalariamittel Lumefantrin ist.

9. Polymer-Lipid-Nanokomplex nach Anspruch 7, Prozess nach einem der Ansprüche 1 bis 5 oder Verfahren nach Anspruch 6, wobei die zumindest eine hydrophobe Wirkstoffverbindung eine Phytochemikalie aus Cannabinoiden, beinhaltend Cannabidiol, ist.

10. Polymer-Lipid-Nanokomplex nach Anspruch 7, Prozess nach einem der Ansprüche 1 bis 5 oder Verfahren nach Anspruch 6, wobei die zumindest eine hydrophobe Wirkstoffverbindung das Anti-Tuberkulose-Arzneimittel Rifampicin ist.

11. Verfahren nach Anspruch 6, wobei die zumindest eine hydrophobe Wirkstoffverbindung zur Verabreichung durch topische, subkutane, intravenöse, intramuskuläre, nasale, sublinguale, bukkale oder ophthalmische Wege formuliert ist.

12. Polymer-Lipid-Nanokomplex nach Anspruch 7 zur Verwendung in einem Verfahren zur Behandlung oder Prophylaxe von Tuberkulose, Malaria, Pilzinfektionen, Virusinfektionen, Angst, Fettleibigkeit, Depression und/oder Schmerzen bei einem Subjekt.

## Revendications

1. Processus permettant la production d'un nanocomplexe polymère-lipide comprenant les étapes de :
I. mélange d'au moins un composé actif hydrophobe positionné sur l'échelle positive de Log P choisi entre Log P 2 et Log P 9 de l'échelle de coefficient de partage, d'un acide gras constitué de l'un quelconque ou de plusieurs de l'acide stéarique, de l'acide palmitique et de l'acide laurique dissous dans un solvant polaire constitué de l'un quelconque de l'éthanol, de l'acétone et d'un mélange d'éthanol et d'acétone, et d'un tensioactif présentant une valeur d'équilibre hydrophile-lipophile (HLB) supérieure à 10 comprenant du polysorbate 80, pour former une phase organique ;
II. éventuellement, chauffage de la phase organique ;
III. distribution de la phase organique dans une solution aqueuse constituée d'un mélange aqueux d'alcool polyvinylique (PVA) et de polyéthylène glycol (PEG) pour former une microémulsion ; et
IV. stabilisation de la microémulsion dans un tampon phosphate à environ 0 °C pour former un nanoprécipité du nanocomplexe polymère-lipide.

2. Processus selon la revendication 1, comprenant en outre une étape finale de séchage du nanoprécipité pour produire une poudre de nanocomplexe polymère-lipide à écoulement libre soit par lyophilisation soit par séchage par pulvérisation.

3. Processus selon l'une des revendications 1 ou 2, comprenant en outre le mélange d'un acide carboxylique organique comprenant l'acide acétique, l'acide lactique, l'acide citrique ou l'acide phosphorique avec la phase organique.

4. Processus selon l'une quelconque des revendications 1 à 3, dans lequel le processus comprend en outre la dissolution d'au moins un polymère ou copolymère biodégradable et biocompatible, de poly(acide lactique-co-glycolique) ou de PLGA, ou d'acide polylactique, d'acide polyglycolique ou de poly ε-caprolactone, dans le solvant polaire avec l'acide gras pour le mélange avec l'au moins un composé actif hydrophobe et le tensioactif afin de former la phase organique.

5. Processus selon l'une quelconque des revendications 1 à 4, dans lequel à l'étape IV, la précipitation de la microémulsion est réalisée par ajout de la microémulsion à la solution tampon phosphate dans un rapport d'environ 1:1.

6. Procédé permettant l'amélioration de la solubilisation aqueuse et l'absorption d'au moins un composé actif hydrophobe, comprenant la formulation de l'au moins un composé actif hydrophobe selon le processus selon l'une quelconque des revendications 1 à 5.

7. Nanocomplexe polymère-lipide obtenu par le processus selon l'une quelconque des revendications 1 à 5.

8. Nanocomplexe polymère-lipide selon la revendication 7, processus selon l'une quelconque des revendications 1 à 5, ou procédé de la revendication 6, dans lequel l'au moins un composé actif hydrophobe est l'agent antipaludique, la luméfantrine.

9. Nanocomplexe polymère-lipide selon la revendication 7, processus selon l'une quelconque des revendications 1 à 5, ou procédé de la revendication 6, dans lequel l'au moins un composé actif hydrophobe est un composé phytochimique issu des cannabinoïdes, y compris le cannabidiol.

10. Nanocomplexe polymère-lipide selon la revendication 7, processus selon l'une quelconque des revendications 1 à 5, ou procédé de la revendication 6, dans lequel l'au moins un composé actif hydrophobe est le médicament antituberculeux, la rifampicine.

11. Procédé selon la revendication 6, dans lequel l'au moins un composé actif hydrophobe est formulé pour une administration par voie topique, sous-cutanée, intraveineuse, intramusculaire, nasale, sublinguale, buccale ou ophtalmique.

12. Nanocomplexe polymère-lipide selon la revendication 7, destiné à être utilisé dans un procédé de traitement ou de prophylaxie de la tuberculose, du paludisme, des infections fongiques, des infections virales, de l'anxiété, de l'obésité, de la dépression et/ou de la douleur chez un sujet.
